# DEMANDE DE BREVET EUROPEEN

(11) **EP 3 912 957 A1**
(43) Date de publication de la demande: **24.11.2021**
(21) Numéro de dépôt: 21176955.9
(22) Date de dépôt: 27.05.2014
(51) Int. Cl.: C01B 21/24, C01B 21/00, A61K 33/00, A61M 16/12

(54) **PROCÉDÉ DE FABRICATION DE MÉLANGES GAZEUX NO/N2 DESTINÉS AU DOMAINE MÉDICAL**

(30) Priorité: 12.06.2013 FR 1355404
(62) Demande divisionnaire de: 14169952.0
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: DE VILLEMEUR, Pierre, 92160 Antony (FR); LECOURT, Laurent, 94250 Gentilly (FR)
(74) Mandataire: Air Liquide

(57) **Abrégé**

L'invention porte sur un procédé de fabrication d'un mélange gazeux NO/N₂ comprenant les étapes d'opérer une purification d'azote gazeux impur contenant des impuretés au moins de type O₂ par mise en contact de l'azote impur avec un catalyseur puis avec un tamis moléculaire ; de mélanger l'azote pur obtenu avec du monoxyde d'azote (NO) ; et d'obtenir un mélange formé de NO et d'azote pur contenant une teneur en NO inférieure à 20% en volume et une proportion d'impuretés de type O₂ inférieure à 5 ppmv et de type H₂O inférieure à 40 ppmv. Le mélange NO/azote ainsi formé peut subir des dilutions supplémentaires avec de l'azote pur. De tels mélanges NO/azote sont utilisables pour traiter par inhalation les vasoconstrictions pulmonaires chez l'adulte ou l'enfant, en particulier chez les nouveaux nés souffrant d'hypertension pulmonaire primitive ou chez les patients subissant une opération de chirurgie cardiaque.

## Description

L'invention concerne un procédé de fabrication d'un mélange gazeux NO/N₂ et son conditionnement subséquent dans un récipient, en particulier une ou plusieurs bouteilles de gaz.

Les mélanges gazeux NO/N₂ sont couramment utilisés pour traiter les vasoconstrictions pulmonaires chez l'adulte, l'enfant, et en particulier chez les nouveaux nés souffrant d'hypertension pulmonaire primitive ou chez les patients subissant une opération de chirurgie cardiaque.

Ces mélanges NO/N₂ sont classiquement conditionnés dans des bouteilles de gaz en acier et aluminium contenant de 100 à 1000 ppm en volume de NO et de l'azote (N₂) pour le reste. Ces bouteilles ont typiquement une contenance en équivalent en eau de 2 à 50 litres.

Le conditionnement, c'est-à-dire la mise en bouteille de ces mélanges, se fait dans des centres de conditionnement de gaz. Pour ce faire, on réalise un ou plusieurs mélanges de NO et d'azote jusqu'à obtenir une concentration en NO dans l'azote désirée.

Cependant, il arrive que les mélanges NO/azote ainsi produits ne soient pas conformes aux spécifications du domaine médical car contenant une quantité trop importante d'impuretés, notamment de type NO₂.

En d'autres termes, la fiabilité des procédés de fabrication de mélanges NO/N₂ classiques est souvent aléatoire.

EP-A-2533125 enseigne un procédé dans lequel un mélange gazeux NO/azote est purifié sur un tamis moléculaire, de type zéolite, gel de silice, alumine ou autre, afin d'éliminer les impuretés de type O₂ et H₂O.

EP-A-240270 propose un procédé de purification d'un gaz inerte, notamment d'azote, par catalyse des impuretés qu'il contient, notamment CO, H₂, O₂... Ce procédé met en œuvre un lit unique pour éliminer toutes les impuretés.

US-A-4,869,883 enseigne également un procédé de purification d'un gaz inerte, notamment d'azote, en trois étapes, comprenant une catalyse oxydative, en présence d'oxygène à teneur élevée, des impuretés qu'il contient, en particulier CO et H₂.

Or, les impuretés NO₂ présentes dans le mélange final NO/azote produit posent un sérieux problème au plan médical car le NO₂ est très toxique lorsqu'il est inhalé par un patient, même à très faibles doses, à savoir de l'ordre de quelques ppm en volume.

Le problème est dès lors de proposer un procédé de fabrication de mélanges gazeux NO/N₂ amélioré permettant d'assurer une bonne précision lors du mélange des composés gazeux mais aussi une fiabilité et une pureté accrues du mélange gazeux NO/N₂ fabriqué puis conditionné, c'est-à-dire de pouvoir produire des mélanges NO/azote exempts ou quasi-exempts d'impuretés NO₂.

La solution de l'invention est alors un procédé de fabrication d'un mélange gazeux NO/N₂ comprenant les étapes de :
a) opérer une purification d'azote gazeux impur contenant des impuretés au moins de type O₂ par :
   i) mise en contact de l'azote impur avec un catalyseur pour éliminer ou convertir au moins une partie des impuretés de type O₂,
   ii) mise en contact de l'azote purifié à la sous-étape i), avec un tamis moléculaire pour éliminer au moins une partie des impuretés de type H₂O issues de l'étape i),
b) mélanger l'azote pur issu de l'étape a) avec du monoxyde d'azote (NO),
c) obtenir un mélange formé de NO et d'azote pur contenant une teneur en NO inférieure à 20% en volume et une proportion d'impuretés de type O₂ inférieure à 5 ppmv et de type H₂O inférieure à 40 ppmv.

Dans le cadre de la présente invention, les pressions données sont des pressions absolues, et les proportions de composés gazeux sont données en % en volume (%v) ou en ppm en volume, i.e. en ppmv.

Selon le cas, le procédé de l'invention peut comprendre l'une ou plusieurs des caractéristiques techniques suivantes :
- à la sous-étape i), on utilise un catalyseur au nickel.
- à la sous-étape ii), on utilise un tamis moléculaire comprenant une zéolite, une alumine ou du gel de silice.
- il comprend une sous-étape iii), dans laquelle on utilise un filtre ayant un diamètre de pore inférieur à 100 µm, de préférence inférieur ou égal à 25 µm.
- la sous-étape iii) est subséquente à la sous-étape ii). Elle permet d'éliminer les particules solides, notamment issues et/ou générées pendant les sous-étapes i) et/ou ii), notamment des particules de type poussières ou résidus résultant d'une attrition du tamis moléculaire.
- en amont de l'étape a), l'azote gazeux impur est obtenu par vaporisation d'azote liquide.
- à l'étape c), on obtient un mélange formé de NO et d'azote pur contenant une proportion d'impuretés de type O₂ inférieure à 1 ppmv et/ou de type H₂O inférieure à 10 ppmv.
- l'étape a) comprend une sous-étape supplémentaire de passage de l'azote purifié à la sous-étape ii) au travers d'au moins un filtre pour éliminer les particules solides, tel que poussières etc...
- l'azote gazeux impur contient moins de 80 ppmv d'impuretés O₂ et/ou moins de 100 ppmv d'impuretés H₂O.
- l'azote gazeux impur contient moins de 50 ppmv d'impuretés O₂ et/ou moins de 67 ppmv d'impuretés H₂O.
- le mélange NO/N₂ obtenu à l'étape c) contient une teneur en NO inférieure ou égale à 10% en volume, de préférence une teneur en NO inférieure ou égale à 8% en volume, de préférence encore une teneur en NO inférieure ou égale à 5% en volume, par exemple de l'ordre de 4 %v.
- à l'étape c), on obtient un mélange formé de NO et d'azote pur contenant une teneur en NO inférieure à 5% en volume et une proportion d'impuretés de type O₂ inférieure à 0,5 ppmv et/ou de type H₂O inférieure à 5 ppmv.
- à l'étape c), on obtient un mélange formé de NO et d'azote pur contenant une teneur en NO inférieure à 4% en volume et une proportion d'impuretés de type O₂ inférieure à 0,1 ppmv et/ou de type H₂O inférieure à 3 ppmv
- le mélange NO/N₂ obtenu à l'étape c) contenant une teneur en NO inférieure ou égale à 20% en volume subit une dilution supplémentaire avec de l'azote de manière à obtenir un mélange final NO/N₂ contenant une teneur en NO inférieure ou égale à 5000 ppmv, de préférence entre 100 et 1000 ppmv.
- le mélange final NO/N₂ contenant une teneur en NO inférieure ou égale à 5000 ppmv, de préférence entre 100 et 1000 ppmv, est conditionné dans un ou plusieurs récipients de stockage de gaz, en particulier des bouteilles de gaz, notamment des bouteilles de gaz ayant une contenance en équivalent en eau de 2 à 50 litres.
- à l'étape c), le mélange gazeux formé de NO et de N₂ contient une teneur en NO inférieure ou égale à 8% en volume.
- à l'étape c), le mélange gazeux formé de NO et de N₂ contient une teneur en NO inférieure ou égale à 5% en volume.
- à l'étape c), le mélange gazeux formé de NO et de N₂ contient une teneur en NO de l'ordre de 4% en volume.
- le mélange gazeux final formé de NO et de N₂ contient par exemple, après dilution avec de l'azote pur, 450 ppmv de NO (+/- 5%), moins de 3 ppmv de NO₂, et de l'azote pour le reste et éventuellement moins de 50 ppmv de N₂O, et/ou moins de 450 ppmv de CO₂.

L'invention va maintenant être mieux comprise grâce à la description donnée ci-après en référence aux Figures annexées parmi lesquelles :
- la Figure 1 schématise un mode de réalisation du procédé selon la présente invention et
- la Figure 2 représente le schéma d'une installation de mise en œuvre du procédé de l'invention.

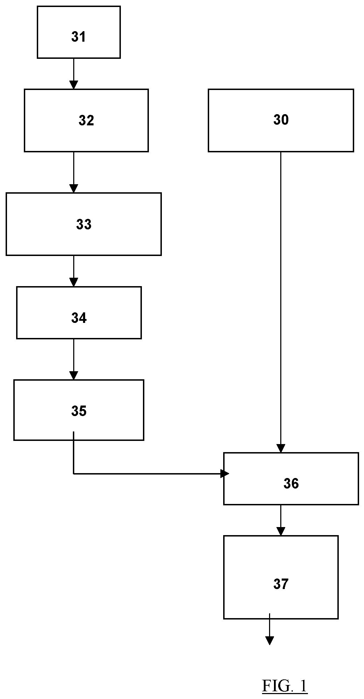

La Figure 1 schématise un mode de réalisation du procédé de fabrication selon la présente invention qui permet de produire des mélanges gazeux NO/N₂ exempts ou quasiment exempts d'impuretés NO₂, par exemple des mélanges gazeux NO/N₂ contenant environ 4% de NO et de l'azote pour le reste.

Selon ce procédé, on procède selon les étapes successives suivantes :
- on fournit (en 31) de l'azote impur ayant la composition suivante : O₂ ≤ 50ppmv, CO ≤ 5ppmv, CO₂ ≤ 300 ppmv, H₂O ≤ 67 ppmv et N₂ pour le reste (env. 99,5 %v)
- on opère une purification de l'azote gazeux impur contenant lesdites impuretés ci-dessus par :
   i) mise en contact (en 32) de l'azote impur avec un catalyseur au nickel par exemple pour éliminer ou convertir au moins une partie des impuretés de type O₂, notamment pour les convertir en vapeur d'eau ou H₂O,
   ii) mise en contact (en 33) de l'azote purifié à la sous-étape i), avec un tamis moléculaire, tel une zéolite 10 A°, pour éliminer au moins une partie des impuretés de type H₂O, soit initialement présentes dans l'azote fourni en 31, soit résultant de la conversion des impuretés O₂ de l'étape i) (en 32),
   iii) on filtre (en 34) l'azote issu de l'étape ii) pour en éliminer les particules solides éventuelles, telles des poussières résultant de l'usure par frottements et attrition des particules de tamis moléculaire lors de l'étape ii),
- on récupère (en 35) de l'azote purifié débarrassé de ses impuretés gazeuses de type O₂ et H₂O, en particulier de l'azote contenant au maximum 200 ppbv d'impuretés O₂, de préférence moins de 100 ppbv de O₂, et au maximum 1 ppmv d'impretés H₂O. L'analyse de ces impuretés est opérée en continu.
- on fournit du monoxyde d'azote ou NO pur (en 30) issu d'une source de NO, telle une capacité de stockage (non montrée),
- on mélange (en 36) l'azote purifié issu de l'étape a) avec le NO pur, par exemple dans un mélangeur de gaz,
- on obtient un mélange formé de NO et d'azote pur contenant une teneur en NO inférieure ou égale à 20%v, typiquement de l'ordre de 4%v, et une proportion de type O₂ inférieure à environ 1 ppmv et de type H₂O inférieure à environ 10 ppmv.

Ensuite, le mélange NO et d'azote pur contenant une teneur en NO inférieure ou égale à 20% en volume, typiquement de l'ordre de 3 à 10% en volume, préférentiellement de l'ordre de 4% en volume, peut subir une ou plusieurs dilutions complémentaires pour diminuer la teneur en NO dans le mélange final, par exemple pour produire un mélange NO/N₂ dépourvu d'impuretés gazeuses de type O₂, H₂O et NO₂, et contenant une proportion de NO comprise entre 100 et 5000 ppmv, typiquement inférieure à 1000 ppmv, qui peut être conditionné en bouteilles de gaz ensuite, comme expliqué ci-après.

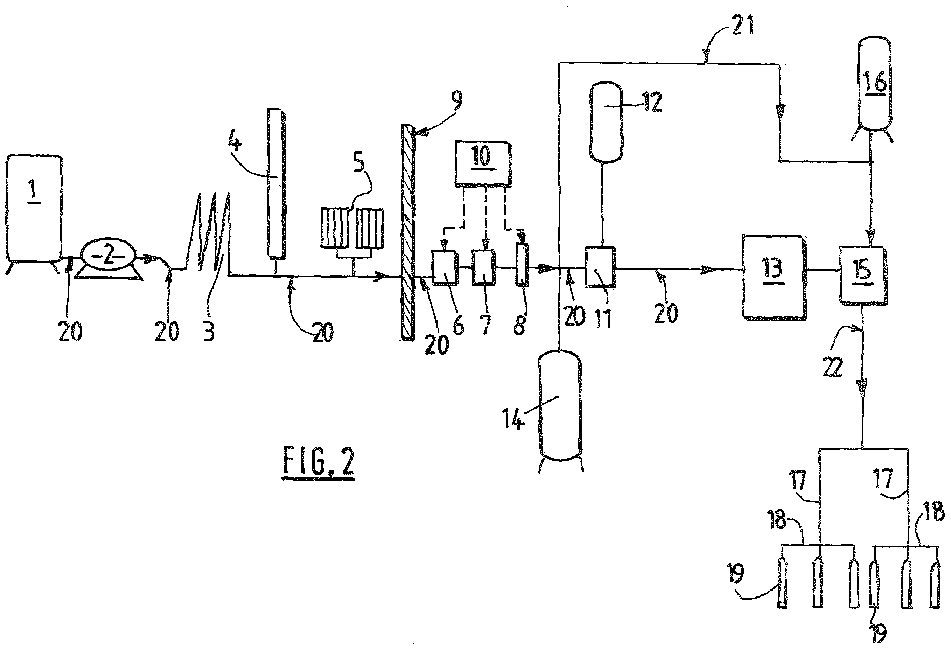

Le procédé selon l'invention illustré en Figure 1 peut être mis en œuvre par le biais d'une installation telle que schématisée en Figure 2.

De l'azote impur contenant au moins des impuretés résiduelles de type O₂ est stocké dans un réservoir 1 sous forme liquide puis soutiré sous forme liquide par une pompe cryogénique 2 qui le comprime à une pression de l'ordre de 100 à 300 bar, avant de l'envoyer vers un réchauffeur atmosphérique 3 où il est vaporisé de manière à obtenir de l'azote gazeux impur.

Comme on le voit, elle comprend une canalisation ou ligne principale 20 permettant de véhiculer l'azote impur depuis le réservoir 1 jusqu'à un réservoir 13 contenant le mélange NO/N₂ à réaliser.

Il est à noter que la ligne 20 peut aussi être reliée fluidiquement à une capacité tampon 4 permettant de stocker une partie de l'azote gazeux impur issu du réchauffeur 3, ainsi qu'à des cadres de secours 5 comprenant chacun plusieurs bouteilles d'azote impur servant de source de N₂ de secours prenant le relai pour fournir de l'azote impur, en cas de dysfonctionnement de la partie amont de l'installation, en particulier de la pompe 2 ou du réchauffeur 3.

Les éléments précédents de l'installation sont préférentiellement situés à l'extérieur d'un bâtiment 9.

En fait, l'azote gazeux impur issu du réchauffeur atmosphérique 3, de la capacité-tampon 4 ou des cadres secours 5 est ensuite véhiculé par la ligne 20 vers un système de purification 6, 7, 8 permettant, selon l'invention, d'éliminer les impuretés O₂ et H₂O qui forment des impuretés résiduelles dans l'azote impur gazeux.

Ces impuretés O₂ et H₂O doivent absolument être éliminées car ce sont elles qui sont responsables de la formation de NO₂ toxique dans le mélange NO/N₂ final stocké dans le réservoir 13. En effet, après mélange de l'azote avec le NO (en 11), les impuretés O₂ et H₂O vont aller réagir avec le NO pour l'oxyder et former ainsi les espèces toxiques de type NO₂.

Donc, en agissant en amont de l'installation, c'est-à-dire avant le mélange du NO avec l'azote qui a lieu dans le mélangeur 11, on peut empêcher ce phénomène et obtenir des mélanges finaux NO/N₂ exempts d'impuretés O₂ et H₂O,et donc également exempts d'espèces toxiques de type NO₂.

Plus précisément, le système de purification 6, 7, 8 comprend, selon l'invention, au moins une enceinte catalytique 6 et au moins un adsorbeur 7, et préférentiellement au moins un compartiment de filtration 8, agencés en série, comme illustré en Figure 2.

Un dispositif de pilotage 10, telle une armoire de commande à automate programmable ou analogue, permet de contrôler le système de purification 6, 7, 8 en agissant notamment sur des vannes (non schématisées) qui contrôlent les entrées et sorties de gaz dans l'enceinte catalytique 6, l'adsorbeur 7, et le compartiment de filtration 8. Ce type de dispositif de pilotage 10 est classique et ne sera pas détaillé ci-après.

Selon un mode de réalisation préféré, le système de purification 6, 7, 8 comprend :
- une enceinte catalytique 6 contenant un catalyseur, de préférence un catalyseur au nickel, permettant de convertir les espèces O₂ présentes dans l'azote impur issu du réchauffeur 3, de la capacité tampon 4 ou des cadres secours 5, en espèces H₂O.
- un (ou plusieurs) adsorbeur 7 contenant au moins un tamis moléculaire, par exemple de type zéolite, gel de silice, alumine ou analogue, ou leurs mélanges. Ce tamis moléculaire permet d'éliminer les espèces H₂O soit présentes dès l'origine dans l'azote impur issu du réservoir 1, soit produites lors de la conversion des impuretés O2 dans l'enceinte catalytique 6.
- un compartiment de filtration 8 comprenant au moins un filtre de diamètre de pore préférentiellement inférieur à 100 µm, typiquement de l'ordre de 25 µm, permettant d'arrêter notamment les résidus solides susceptibles d'être libérés au sein de l'adsorbeur 7 du fait de l'attrition des particules de tamis moléculaire sous l'effet des variations de pression et des débits de gaz traité.

L'adsorbeur ou les adsorbeurs 7 peuvent fonctionner en mode PSA (adsorption à pression modulée), en mode VSA (adsorption à vide modulée) ou en mode TSA (adsorption à température modulée) par exemple, donc avec phases de régénération classiques par variation de pression, dépression ou température. Ce type de procédé d'adsorption est connu de l'homme du métier et ne nécessite pas d'être détaillé dans le cadre de la présente invention.

De même, l'enceinte catalytique 6 peut être périodiquement régénérée par un gaz chaud par exemple. Là encore, ce type de procédé de catalyse et régénération est connu de l'homme du métier et ne nécessite pas d'être détaillé dans le cadre de la présente invention.

Il est à noter par ailleurs que d'autres catalyseurs sont susceptibles de convenir également, par exemple des catalyseurs au platine ou palladium. De même, le tamis moléculaire peut être un type non-zéolitique, par exemple de l'alumine activée ou du gel de silice. Un homme du métier est capable via des tests de routine de sélectionner le ou les catalyseurs et les adsorbants les plus adaptés, ainsi qu'un ou plusieurs filtres adéquats.

Une fois débarrassé de ses impuretés, l'azote purifié peut être stocké dans un réservoir de stockage 14 ou être convoyé directement par la ligne 20 jusqu'à un dispositif de mélange ou mélangeur 11, lequel est également alimenté par une source de NO 12 pur, de manière à y opérer le mélange de NO et d'azote purifié dans les proportions désirées, par exemple un mélange NO/N₂ contenant environ 4% de NO.

Ce mélange de NO et d'azote purifié peut ensuite être stocké dans un second réservoir de stockage 13 avant d'être envoyé, via une ligne d'alimentation des rampes 22 et des lignes secondaires de gaz 17 se ramifiant à partir de la ligne d'alimentation des rampes 22, vers des rampes de conditionnement 18 où il peut être mis en bouteilles de gaz 19.

Toutefois, avant de réaliser le conditionnement du mélange NO/N₂ en bouteilles 19 de gaz, il peut être utile, voire nécessaire, d'ajuster la teneur en NO, en particulier pour la réduire à une teneur finale d'utilisation désirée, par exemple une teneur finale de NO comprise entre 100 et 5000 ppmv, typiquement moins de 1000 ppmv.

Pour ce faire, on peut prévoir de réaliser une dilution supplémentaire du mélange NO/N₂ au sein d'un second dispositif mélangeur 15 avec de l'azote pur, de manière à réduire la teneur en NO dans le mélange NO/N₂ et obtenir ainsi un mélange final NO/N₂ contenant entre 200 et 5000 ppmv de NO, le reste étant de l'azote, typiquement entre 200 et 1000 ppmv.

Le second dispositif mélangeur 15 est agencé en aval du premier mélangeur 11 et du réservoir de stockage 13. Il est alimenté par ailleurs par de l'azote pur servant à la dilution du mélange NO/N₂ et issu d'une capacité de stockage 16 qui peut être alimentée par de l'azote purifié convoyé par une ligne de dérivation 21 venant se raccorder à la ligne principale 20, en aval du système de purification 6, 7, 8, comme illustré en Figure 2.

Les bouteilles de gaz 19 sont du type à corps en acier, en aluminium ou en alliage d'aluminium, et ont une contenance (équivalent en eau) comprise entre 2 et 50 litres environ. Le mélange gazeux y est stocké à une pression allant jusqu'à 300 bar.

Il est à noter que des débitmètres (non montrés) peuvent être prévus pour mesurer la quantité de N₂ et de NO circulant dans les lignes d'amenée de gaz, notamment la ligne principale 20, la ligne de dérivation 21 ou la ligne d'alimentation des rampes 22, et de transmettre les informations mesurées au dispositif de pilotage 10 ou à tout autre dispositif de contrôle de l'installation, tel un ordinateur ou analogue.

### Exemple

Une installation semblable à celle de la Figure 2 a été utilisée pour mettre en œuvre le procédé de la Figure 1 de manière à tester l'efficacité de la solution proposée par la présente invention, dans la fabrication d'un mélange gazeux NO/N₂ contenant environ 4% de NO et dépourvus d'impuretés toxiques de type NO₂.

Les conditions du procédé sont données dans le Tableau 1 ci-après.

**Tableau 1**

| | |
|---|---|
| Teneur initiale en O₂ dans l'azote impur | ≤ 50 ppmv |
| Teneur initiale en H₂O dans l'azote impur | ≤67 ppmv |
| Catalyseur Ni-1404 T 3/16" | nickel (68%) |
| Tamis moléculaire (adsorbant) | Zéolite 10 A° |
| Type de filtre (diamètre de pores) | 25 µm |
| Teneur en O₂ dans l'azote purifié | < 0,1 ppmv env. |
| Teneur en H₂O dans l'azote purifié | < 3 ppmv env. |
| Mélange NO/N₂ obtenu | NO : 3,8 à 4,2 % vol. |
| | NO₂ : env. 0 ppmv |
| | Azote : reste |
| Mélange NO/N₂ final après dilution supplémentaire avec de l'azote pur (plusieurs mélanges différents ont été réalisés) | NO : 200 à 1000 ppmv |
| | NO₂ : env. 0 ppmv |
| | Azote : reste |

Le Tableau 2 ci-après donne les caractéristiques du catalyseur utilisé pour arrêter les impuretés de type O₂ et les convertir en H₂O notamment.

**Tableau 2**

| Nom du catalyseur | Ni-1404 T 3/16" |
|---|---|
| Fabricant | Engelhard |
| Métal du catalyseur | Nickel |
| Teneur en nickel | 68% en poids |
| Aire de surface | 130 m²/g |
| Volume de pore total | 0,38 cc/g |
| Résistance à l'écrasement latéral (*Side crush strength*) | 7,5 kgs |
| Rapport de valeur réduit (*Reduced value ratio*) | 0,55 |
| Type de catalyseur | hydrogénation |
| Forme du catalyseur | particules |

Le Tableau 3 ci-après donne les caractéristiques du tamis moléculaire utilisé pour arrêter les impuretés de type H₂O (vapeur d'eau).

**Tableau 3**

| Tamis moléculaire | zéolite |
|---|---|
| Fabricant | Grace |
| Type | 544 |
| Taille de pore | 10 A° |
| Granulométrie | 1,6-2,5 mm |
| Densité apparente | 670 g/l |
| Capacité d'adsorption en eau | 22 % en poids |
| Forme de l'adsorbant | particules |

Le filtre utilisé pour arrêter les particules solides, par exemple les poussières résultant de l'attrition du tamis moléculaire, a un diamètre de pores moyen de l'ordre de 25 µm.

Au vu des résultats donnés dans le Tableau 1, on constate que le procédé de l'invention est efficace puisqu'il permet d'éliminer la majeure partie des impuretés O₂ et H₂O susceptibles d'être présentes dans l'azote impur et ainsi d'obtenir un mélange NO/N₂ contenant une proportion finale de H₂O inférieure à environ 3 ppmv et une proportion finale de O₂ inférieure à environ 0,1 ppmv, conduisant à une formation quasi nulle de NO₂ toxique, dans le mélange final.

Les mélanges gazeux NO/N₂ ainsi produits peuvent être utilisés pour traiter par inhalation les vasoconstrictions pulmonaires chez l'adulte ou l'enfant, en particulier chez les nouveaux nés souffrant d'hypertension pulmonaire primitive ou chez les patients subissant une opération de chirurgie cardiaque.

## Revendications

1. Mélange gazeux NO/N₂ formé de NO et d'azote (N₂) contenant une teneur en NO inférieure à 20% en volume et une proportion d'impuretés de type O₂ inférieure à 5 ppmv et de type H₂O inférieure à 40 ppmv obtenu par un procédé de fabrication d'un mélange gazeux NO/N₂ comprenant les étapes de :
a) opérer une purification d'azote gazeux impur contenant des impuretés au moins de type O₂ par :
i) mise en contact de l'azote impur avec un catalyseur pour éliminer ou convertir au moins une partie des impuretés de type O₂,
ii) mise en contact de l'azote purifié à la sous-étape i), avec un tamis moléculaire pour éliminer au moins une partie des impuretés de type H₂O issues de l'étape i),
b) mélanger l'azote pur issu de l'étape a) avec du monoxyde d'azote (NO),
c) obtenir ledit mélange NO/N₂,
pour une utilisation par inhalation pour traiter des vasoconstrictions pulmonaires chez un adulte ou un enfant.

2. Mélange gazeux pour une utilisation selon la revendication 1, **caractérisé en ce qu'**il contient une proportion d'impuretés de type O₂ inférieure à 1 ppmv et/ou de type H₂O inférieure à 10 ppmv.

3. Mélange gazeux pour une utilisation selon l'une des revendications précédentes, **caractérisé en ce qu'**il contient une teneur en NO inférieure ou égale à 10% en volume.

4. Mélange gazeux pour une utilisation selon l'une des revendications précédentes, **caractérisé en ce qu'**il contient une teneur en NO inférieure ou égale à 8% en volume.

5. Mélange gazeux pour une utilisation selon l'une des revendications précédentes, **caractérisé en ce qu'**il contient une teneur en NO inférieure ou égale à 5% en volume.

6. Mélange gazeux pour une utilisation selon l'une des revendications précédentes, **caractérisé en ce qu'**il contient une teneur en NO inférieure à 5% en volume et une proportion d'impuretés de type O₂ inférieure à 0,5 ppmv et/ou de type H₂O inférieure à 5 ppmv.

7. Mélange gazeux pour une utilisation selon l'une des revendications précédentes, **caractérisé en ce qu'**il contient une teneur en NO inférieure à 4% en volume et une proportion d'impuretés de type O₂ inférieure à 0,1 ppmv et/ou de type H₂O inférieure à 3 ppmv.

8. Mélange gazeux pour une utilisation selon l'une des revendications précédentes, **caractérisé en ce que** le mélange NO/N₂ obtenu à l'étape c) contenant une teneur en NO inférieure ou égale à 20% en volume subit une dilution supplémentaire avec de l'azote de manière à obtenir un mélange final NO/N₂ contenant une teneur en NO inférieure ou égale à 5000 ppmv.

9. Mélange gazeux pour une utilisation selon la revendication 8, **caractérisé en ce que** le mélange final NO/N₂ contient une teneur en NO comprise entre 100 et 1000 ppmv.

10. Mélange gazeux pour une utilisation selon l'une des revendications 8 ou 9, **caractérisé en ce qu'**il est conditionné dans un ou plusieurs récipients de stockage de gaz.

11. Mélange gazeux pour une utilisation selon la revendication 10, **caractérisé en ce qu'**il est conditionné dans une ou des bouteilles de gaz (19).

12. Mélange gazeux pour une utilisation selon la revendication 11, caractérisé en que les bouteilles de gaz ont une contenance en équivalent en eau de 2 à 50 litres.

13. Mélange gazeux pour une utilisation selon la revendication 1, pour traiter un nouveau-né souffrant d'hypertension pulmonaire primitive (PPHN).

14. Mélange gazeux pour une utilisation selon la revendication 1, pour traiter un patient en chirurgie cardiaque.
